Europäisches Patentamt

European Patent Office

Office européen des brevets

(19)

(11) Veröffentlichungsnummer: **0 137 983**
**B1**

# (12) EUROPÄISCHE PATENTSCHRIFT

(45) Veröffentlichungstag der Patentschrift:
08.04.87

(21) Anmeldenummer: 84110095.1

(22) Anmeldetag: 24.08.84

(51) Int. Cl.⁴: **C 07 C 143/11, C 07 C 139/00**

(54) Ethersulfonate und ihre Herstellung.

(30) Priorität: 01.09.83 DE 3331513

(43) Veröffentlichungstag der Anmeldung:
24.04.85 Patentblatt 85/17

(45) Bekanntmachung des Hinweises auf die Patenterteilung:
08.04.87 Patentblatt 87/15

(84) Benannte Vertragsstaaten:
AT BE CH DE FR GB IT LI LU NL SE

(56) Entgegenhaltungen:
EP-A-0 064 384
CH-A-172 565
DD-A-91 020
DE-A-2 949 694
DE-B-1 081 172
DE-C-1 075 779
GB-A-1 539 001

(73) Patentinhaber: **Henkel Kommanditgesellschaft auf Aktien, Postfach 1100 Henkelstrasse 67, D-4000 Düsseldorf- Holthausen (DE)**

(72) Erfinder: **Piorr, Norbert, Dr., Kieselei 12, D-4030 Ratingen- Hösel (DE)**
Erfinder: **Meffert, Alfred, Dr., Marie- Curie- Strasse 10, D-4019 Monheim (DE)**

EP 0 137 983 B1

LIBER, STOCKHOLM 1987

## Beschreibung

Gegenstand der Erfindung ist ein Verfahren zur Herstellung von Ethersulfonaten. Ein weiterer Gegenstand der Erfindung sind Ethersulfonate, wie sie nach dem neuen Verfahren erhalten werden.

Unter den zahlreichen Gruppen anionischer Tenside kommt denjenigen, die eine -$SO_3^\ominus$-Gruppe als wasserlöslich machende Gruppe enthalten, die größte technische Bedeutung zu. Innerhalb dieser Gruppe werden die Sulfattenside und die Sulfonattenside unterschieden.

Die Sulfattenside sind Halbestersalze der Schwefelsäure. Von diesen kommt den Alkylethersulfaten besondere Wichtigkeit zu, da sie wegen der Glycolethergruppen im Molekül über eine sehr gute Wasserlöslichkeit verfügen und daher besonders zur Herstellung flüssiger Wasch- und Reinigungsmittel gut geeignet sind.

Nachteilig bei den Alkylethersulfaten ist die mangelhafte Hydrolysestabilität der Verbindungen, die es nicht zuläßt, die Produkte für saure Reinigungsmittel zu verwenden.

Die Sulfonattenside sind Salze von Alkylsulfonsäuren und sind auch im sauren Medium gegen Hydrolyse stabil.

Es gibt aber nur wenige, technisch aufwendige Verfahren zur Herstellung von Sulfonattensiden, die zur Steigerung der Wasserlöslichkeit Glycolethergruppen enthalten. Beispiele für bekannte Ethersulfonate sind die aus US-PS 1.985.747 bekannten Alkylglycolethersulfonate und die aus DE-AS 1.075.779 und DE-AS 1.081.172 bekannten Alkylglycerylethersulfonate.

Aus GB-A 1 539 001 war die Umsetzung von Hydroxylverbindungen mit z. B. Natriumisethionat zu primären Ethersulfonaten bekannt, bei der jedoch nur unbefriedigende Umsetzungsgrade erreicht werden. Aus EP-A 64 354 weren Alkoxypolyethoxypropansulfonate bekannt, die aus Alkylpolyglykolethern durch Überführung in die Allylether und Umsetzung mit Natriumsulfit erhalten werden.

Es bestand daher die Aufgabe, ein Verfahren zur Herstellung von Ethersulfonattensiden zu finden, welches solche Produkte aus leicht zugänglichen Rohstoffen nach einem einfachen, kontinuierlich durchzuführenden Sulfonierungsverfahren zugänglich macht.

Diese Aufgabe wurde erfindungsgemäß gelöst durch ein Verfahren zur Herstellung von Ethersulfonaten, welches dadurch gekennzeichnet ist, daß man einen ungesättigten Fettalkyl-niedrigalkylether oder einen ungesättigten Fettalkylpolyoxyalkyl-niedrigalkylether der allgemeinen Formel I

$$R - O(C_nH_{2n}O)_x - R^1 \quad I$$

in welcher R einen Oleylrest oder einen überwiegend aus Oleyl-, Palmitoleyl- und Linoleylresten bestehenden Fettalkylrest, n eine ganze Zahl von 2 - 4, $X = 0$ oder eine Zahl bis 30 und $R^1$ einen Alkylrest mit 1 - 6 C-Atomen bedeutet, mit Schwefeltrioxid umsetzt, das Umsetzungsprodukt in die wäßrige Lösung von 1 - 1,2 Mol Alkalihydroxid pro Mol angelagertes SO einträgt und die Lösung bis zur Hydrolyse gebildeter Sultone erwärmt.

Ethersulfonate mit besonders günstigen Anwendungseigenschaften werden erhalten, wenn zur Sulfonierung ein ungesättigter Fettalkylpolyoxyalkyl-niedrigalkylether eingesetzt wird, der 3 - 20 Ethylenglycolethergruppen enthält, d. h. daß in der allgemeinen Formel I $n = 2$ und x eine Zahl von 3 - 20 ist. Der Rest $R^1$ ist bevorzugt ein n-Butylrest. Die Sulfonierung dieser ungesättigten Fettalkyl(polyoxyalkyl)-niedrigalkylether wird bevorzugt mit gasförmigem Schwefeltrioxid bei Temperaturen von 20 - 100°C durchgeführt. Diese Umsetzung läßt sich in üblichen, für die Sulfonierung von Fettalkoholen, Fettsäureestern, Alkylbenzol oder Olefinen geeigneten Reaktoren, bevorzugt vom Typ der Fallfilmreaktoren, kontinuierlich durchführen.

Das Schwefeltrioxid wird dabei mit Luft oder Stickstoff verdünnt, bevorzugt in Form eines Gasgemisches mit ca. 1 - 10 Vol% $SO_3$ mit dem ungesättigten Fettalkyl(polyoxyalkyl)-niedrigalkylether, bevorzugt bei einer Temperatur von 50 - 70° C, in Berührung gebracht.

Das rohe Sulfonierungsprodukt wird in eine wäßrige Lösung eines Alkalihydroxids eingeleitet, wobei dieses in einer Menge von 1 - 1,2 Mol Alkalihydroxid pro Mol angelagerten Schwefeltrioxids vorliegen sollte. Der leichte Überschuß an Alkalihydroxid dient der Neutralisation des im Sulfonierungsprodukt gelösten gasförmigen $SO_3$ und der Aufrechterhaltung eines Alkaliüberschusses, der die anschließende Hydrolysestufe begünstigt. Als Alkalihydroxid wird bevorzugt Natriumhydroxid eingesetzt. Die Konzentration der Alkalihydroxidlösung wird so gewählt, daß das Endprodukt eine niedrigviskose Lösung bildet.

Das Reaktionsprodukt enthält neben ungesättigten Ethersulfonsäuren auch Sultone. Die Bildung von Sultonen bei der Sulfonierung von olefinischen Doppelbindungen ist eine an sich bekannte Nebenreaktion, die auch bei dem erfindungsgemäßen Verfahren auftritt. Zur Überführung der im Reaktionsprodukt unerwünschten Sultone in ungesättigte Sulfonate und Hydroxysulfonate ist es erforderlich, die wäßrige Lösung einer Hydrolysestufe zu unterwerfen.

Die Hydrolyse wird durch Erwärmen der Lösung bis zur völligen Zerstörung der Sultone durchgeführt. Die dafür erforderliche Zeit ist von den Hydrolysebedingungen abhängig. Sie läßt sich z. B. bei Siedetemperatur unter Normaldruck in 4 - 6 Stunden, bei höherer Temperatur unter Druck aber in erheblich kürzerer Zeit bewerkstelligen.

Die Ethersulfonate fallen nach dem erfindungsgemäßen Verfahren in Form von dunkel- bis hellgelben wäßrigen alkalische Lösungen an. Sie können, wenn dies gewünscht wird, mit Wasserstoffperoxidlösung oder Chlorlauge in bekannter Weise gebleicht werden. Der pH-Wert der Lösungen kann unter Verwendung von z. B. Phosphorsäure, Citronensäure oder Milchsäure neutral eingestellt werden. Zur Stabilisierung gegen Bakterienbefall empfiehlt sich eine Konservierung mit Formaldehydlösung, p-Hydroxybenzoat, Sorbinsäure oder anderen bekannten Konservierungsstoffen.

Die Ausgangsprodukte der allgemeinen Formel I sind nach literaturbekannten Verfahren zugänglich. Ihre Herstellung geht aus von ungesättigten Fettalkoholen, z. B. von Oleylalkohol oder technischen, überwiegend aus Oleylalkohol, Palmitoleylalkohol und Linoleylalkohol bestehenden Alkoholschnitten. Geringe Anteile an gesättigten Alkoholen, z. B. an Cetyl- und Stearylalkohol sind tragbar, vor allem wenn die daraus durch Oxalkylierung und Veretherung hergestellten Produkte der allgemeinen Formel I selbst wasserlöslich sind. Geeignete ungesättigte Alkohole sind durch Hydrierung von Ölsäure oder von technischen Oleinsäuren herstellbar und im Handel erhältlich. Bevorzugt werden technische Cetyl-Oleyl- und Oleyl-Linoleylalkoholschnitte mit einer Jodzahl im Bereich von 70 - 130 eingesetzt.

Die Oxalkylierung ungesättigter Alkohole mit Ethylenoxid, Propylenoxid, Butylenoxid oder Gemischen dieser Alkylenoxide ist ein seit langem großtechnisch durchgeführtes Verfahren. Dabei werden Gemische homologer Oxalkylate erhalten, deren mittlerer Oxalkylierungsgrad der Menge des angelagerten Alkylenoxids entspricht. Die Veretherung der endständigen Hydroxylgruppe der ungesättigten Alkohole und/oder deren Oxalkylate erfolgt nach ebenfalls literaturbekannten Methoden. Sie kann z. B. in der Weise durchgeführt werden, daß der Alkohol oder das Oxalkylat mit einem Alkalimetall in das Alkoholat überführt wird und dieses mit einem Alkylhalogenid zur Umsetzung gebracht wird. Ein anderes Verfahren besteht darin, daß der Alkohol oder das Oxalkylat mit einem Alkylhalogenid in Gegenwart eines feinpulverisierten Alkalimetallhydroxids bei erhöhter Temperatur umgesetzt wird. Schließlich kann das Oxalkylat mit einem Alkylchloridoder Alkylbromid der allgemeinen Formel $R^1$ - Cl oder $R^1$-Br, worin Rl eine Niedrigalkylgruppe mit 1 - 6 C-Atomen darstellt, in Gegenwart einer wäßrigen Lösung von NaOH oder KOH gemäß DE-PS 2 800 710 zur Umsetzung gebracht werden.

Die nach dem erfindungsgemäßen Verfahren zugänglichen Ethersulfonate weisen eine hohe Oberflächenaktivität und gute anwendungstechnische Tensideigenschaften auf.

Besonders günstig ist das Benetzungsvermögen gegenüber Glas, Textil und Polyester. Die gute Wasserlöslichkeit und das befriedigende Emulgiervermögen lassen die Produkte sowohl als technische Netzmittel als auch für die Anwendung in Wasch- und Reinigungsmitteln geeignet erscheinen. Besonders hervorzuheben ist die Hydrolysestabilität auch im sauren Medium, was den Anwendungsbereich z. B. gegenüber den Ethersulfaten erheblich ausweitet.

Die folgenden Beispiele sollen den Erfindungsgegenstand näher erläutern:

## Beispiele

1. Oleylalkohol-5EO-butylethersulfonat, Na-Salz

885 g (ca. 1.5 Mol) des n-Butylethers eines Anlagerungsproduktes von 5 Mol Ethylenoxid an einen technischen Oleylalkohol der Jodzahl 94 (HD-Ocenol® 92/95) wurden mit 187,8 g (ca. 2.35 Mol) gasförmigem (aus Oleum ausgetriebenem) $SO_3$ bei 60°C in einem Fallfilmreaktor kontinuierlich umgesetzt. Das Reaktionsprodukt wurde nach dem Austritt aus dem Reaktor in eine Lösung von 98 g (2,45 Mol) NaOH in 1600 g Wasser eingetragen. Die dabei erhaltene Lösung wurde 6 Stunden unter Rückfluß zum Sieden erhitzt. Dabei wurde eine Lösung des Ethersulfonats mit folgenden Kenndaten erhalten:
Aktivsubstanz (Trockenrückstand): 43,0 Gew.-%
Aniontensid (DGF-Methode H-III-10): 0,52 mval/g ·
$Na_2SO_4$: 2,0 Gew.-%
Unsulfiertes (DGF-Methode G-III-6 b): 6,0 Gew.-%

2. Oleylalkohol-10EO-butylether-sulfonat, Na-Salz

Nach dem Verfahren gemäß Beispiel 1 wurde der n-Butyl-ether eines Anlagerungsprodukts von 10 Mol Ethylenoxid an einen technischen Oleylalkohol (HD-Ocenol® 92/95) sulfoniert. Es wurde ein Sulfonat mit den folgenden Kenndaten erhalten:
Aktivsubstanz (Trockenrückstand): 33,8 Gew.-%
Aniontensid (DGF-Methode H-III-10): 0,39 mval/g
$Na_2SO_4$: 1,9 Gew.-%
Unsulfiertes (DGP-Methode GIII-6b): 4,8 Gew.-%

3. Oleylalkohol-20EO-butylether-sulfonat, Na-Salz

Nach dem gleichen Verfahren wie in Beispiel 1 wurde ausgehend von dem n-Butylether eines Anlagerungsproduktes von 20 Mol Ethylenoxid an einen technischen Oleylalkohol (HD-Ocenol® 92/96) das entsprechende Sulfonat mit folgenden Kenndaten erhalten:
Aktivsubstanz (Trockenrückstand) 37,8 Gew.-%
Aniontensid (DGF-Methode H-III-10): 0,32 mval/g
$Na_2SO_4$: 1,4 Gew.-%
Unsulfiertes (DGF-Methode G III-6 b): 6,6 Gew.-%

# 0 137 983

## Patentansprüche

1. Verfahren zur Herstellung von Ethersulfonaten, dadurch gekennzeichnet, daß man einen ungesättigten Fettalkyl- oder Fettalkylpolyoxyalkyl-niedrigalkylether der allgemeinen Formel I

$R - O-(C_nH_{2n}O)_x-R^1$

in welcher R einen Oleylrest oder einen überwiegend aus Oleyl-, Palmitoleyl- und Linoleylresten bestehenden Fettalkylrest, n eine ganze Zahl von 2 - 4, X = 0 oder eine Zahl bis 30 und $R^1$ einen-Alkylrest mit 1 - 6 C-Atomen bedeutet, mit Schwefeltrioxid umsetzt, das Umsetrzungsprodukt in die wässrige Lösung von 1 - 1,2 Mol Alkalihydroxid pro Mol angelagertes $SO_3$ einträgt und die Lösung bis zur Hydrolyse gebildeter Sultone erwärmt.

2. Verfahren nach Anspruch 1, dadurch gekennzeichnet, daß n = 2 und x eine Zahl von 3 - 20 ist.

3. Verfahren nach Anspruch 1 oder 2, dadurch gekennzeichnet, daß $R^1$ ein n-Butylrest ist.

4. Verfahren nach Anspruch 1 - 3, dadurch gekennzeichnet, daß als Alkalihydroxid Natriumhydroxid eingesetzt wird.

5. Ethersulfonate und deren Alkalisalze, dadurch erhältlich, daß man einen ungesättigten Fettalkyl- oder Fettalkylpolyoxyalkyl-niedrigalkylether der allgemeinen Formel I

$R - O-(C_nH_{2n}O)_x-R^1$ I

in welcher R einen Oleylrest oder einen überwiegend aus Oleyl-, Palmitoleyl- und Linoleylresten bestehenden Fettalkylrest, n eine ganze Zahl von 2 - 4, X = 0 oder eine Zahl bis 30 und $R^1$ einen Alkylrest mit 1 - 6 C-Atomen bedeutet, mit Schwefeltrioxid umsetzt, das Umsetrzungsprodukt in die wässrige Lösung von 1 - 1,2 Mol Alkalihydroxid pro Mol angelagertes $SO_3$ einträgt und die Lösung bis zur Hydrolyse gebildeter Sultone erwärmt.

6. Ethersulfonate und deren Alkalisalze nach Anspruch 5, dadurch gekennzeichnet, daß n = 2 und x eine Zahl von 3 - 20 ist.

7. Ethersulfonate und deren Alkalisalze nach Anspruch 5 oder 6, dadurch gekennzeichnet, daß $R^1$ ein n-Butylrest ist.

8. Ethersulfonate und deren Alkalisalze nach Anspruch 5 - 7 dadurch gekennzeichnet, daß als Alkalihydroxid Natriumhydroxid eingesetzt wird.

9. Verwendung von Ethersulfonaten nach Anspruch 5 - 8 zur Herstellung von Wasch- und Reinigungsmitteln.


## Claims

1. A process for the production of ester sulfonates, characterized in that an unsaturated fatty alkyl or fatty alkyl-polyoxyalkyl lower alkyl ether corresponding to the following general formula

$R-O-(C_nH_{2n}O)_x-R^1$ (I)

in which R is an oleyl radical or a fatty alkyl radical consisting predominantly of oleyl, palmitoleyl and linoleyl radicals, n is an integer of from 2 to 4, X = 0 or a number up to 30 and $R^1$ is a $C_1-C_6$ alkyl radical, is reacted with sulfur trioxide, the reaction product is introduced into an aqueous solution of 1 to 1.2 moles alkali hydroxide per mole added $SO_3$ and the solution is heated until the sultones formed have been hydrolyzed.

2. A process as claimed in Claim 1, characterized in that n = 2 and x is a number of from 3 to 20.

3. A process as claimed in Claim 1 or 2, characterized in that $R^1$ is an n-butyl radical.

4. A process as claimed in Claims 1 to 3, characterized in that sodium hydroxide is used as the alkali hydroxide.

5. Ether sulfonates and alkali salts thereof obtainable by reacting an unsaturated fatty alkyl or fatty alkylpolyoxyalkyl lower alkyl ether corresponding to the following general formula

$R-O-(C_nH_{2n}O)_x-R^1$ I

in which R is an oleyl radical or a fatty alkyl radical consisting predominantly of oleyl, palmitoleyl and linoleyl radicals, n is an integer of from 2 to 4, X = 0 or a number up to 30 and $R^1$ is a $C_1-C_6$ alkyl radical, with sulfur trioxide, introducing the reaction product into an aqueous solution of 1 to 1.2 moles alkali hydroxide per mole added $SO_3$ and heating the solution until the sultones formed have been hydrolyzed.

6. Ether sulfonates and alkali salts thereof, as claimed in claim 5 characterized in that n = 2 and x is a number of from 3 to 20.

7. Ether sulfonates and alkali salts thereof, as claimed in claim 5 or 6 characterized in that $R^1$ is an n-butyl radical.

8. Ether sulfonates and alkali salts thereof as claimed in Claims 5 to 7, characterized in that sodium hydroxide is used as the alkali hydroxide.

9. The use of the ether sulfonates claimed in Claims 5 to 8 for the production of detergents.

4

## Revendications

1. Procédé de fabrication d'éther-sulfonates, caractérisé en ce qu'on fait réagir un alcoyl gras- ou un alcoylgras-polyoxyalcoyl-alcoyl inférieur-éther non saturé de formule générale I:

$R - O - (C_nH_{2n}O)_x - R^1$ I

dans laquelle R signifie un radical oléyle ou un radical alcoyle gras consistant principalement en des radicaux oléyle, palmitoléyle et linoléyle, n un nombre entier de 2 à 4, X = 0 ou un nombre allant jusqu'à 30 et $R^1$ un radical alcoyle ayant 1 à 6 atomes de carbone, avec du trioxyde de soufre, en ce qu'on introduit le produit de réaction dans la solution aqueuse de 1 à 1,2 mole d'hydroxyde alcalin par mole de $SO_3$ fixé et en ce qu'on chauffe la solution jusqu'à hydrolyse des sultones formées.

2. Procédé selon la revendication 1, caractérisé en ce que n = 2 et x un nombre de 3 à 20.

3. Procédé selon la revendication 1 ou 2, caractérisé en ce que $R_1$ est un radical n-butyle.

4. Procédé selon les revendications 1 à 3, caractérisé en ce qu'on utilise de l'hydroxyde de sodium en tant qu'hydroxyde alcalin.

5. Ether-sulfonates et leurs sels alcalins qui sont obtenus quand on fait réagir un alcoyl gras- ou alcoyl gras-polyoxyalcoyl-alcoyl inférieuréther non saturé de formule générale I:

$R - O - (C_nH_{2n}O)_x - R^1$ I

dans laquelle R représente un radical oléyle ou un radical alcoyle gras consistant principalement en radicaux oléyle, palmitoléyle et linoléyle, n un nombre entier de 2 à 4, X = 0 ou un nombre allant jusqu'à 30 et $R^1$ un radical alcoyle ayant 1 à 6 atomes de carbone, avec du trioxyde de soufre, en ce qu'on introduit le produit de réaction dans la solution aqueuse de 1 à 1,2 mole d'hydroxyde alcalin par mole de $SO_3$ fixé et en ce qu'on chauffe la solution jusqu'à hydrolyse des sultones formées.

6. Ether-sulfonates et leurs sels alcalins selon la revendication 5, caractérisés en ce que n = 2 et X un nombre de 3 à 20.

7. Ether-sulfonates et leurs sels alcalins selon la revendication 5 ou 6, caractérisés en ce que $R^1$ est un radical n-butyle.

8. Ether-sulfonates et leurs sels alcalins selon les revendications 5 à 7, caractérisés en ce qu'on utilise de l'hydroxyde de sodium en tant qu'hydroxyde alcalin.

9. Utilisation des éther-sulfonates selon les revendications 5 à 8 pour la fabrication d'agents de lavage et de nettoyage.